(19) Europäisches Patentamt
European Patent Office
Office européen des brevets

(11) **EP 3 376 505 A1**

# (12) EUROPEAN PATENT APPLICATION

(43) Date of publication:
**19.09.2018 Bulletin 2018/38**

(51) Int Cl.:
**G16H 50/20** (2018.01)     **A61B 5/00** (2006.01)
**G01N 33/497** (2006.01)

(21) Application number: **18161519.6**

(22) Date of filing: **13.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2017   JP 2017050417**

(71) Applicant: **Tanita Corporation**
**Tokyo 174-8630 (JP)**

(72) Inventors:
• **KODAMA, Miyuki**
**Itabashi-ku, Tokyo 174-8630 (JP)**

• **KUSAMA, Ayumi**
**Itabashi-ku, Tokyo 174-8630 (JP)**
• **MOCHIZUKI, Kei**
**Itabashi-ku, Tokyo 174-8630 (JP)**
• **KUMEKAWA, Mayumi**
**Itabashi-ku, Tokyo 174-8630 (JP)**
• **KANARI, Shinji**
**Itabashi-ku, Tokyo 174-8630 (JP)**

(74) Representative: **Grünecker Patent- und Rechtsanwälte**
**PartG mbB**
**Leopoldstraße 4**
**80802 München (DE)**

(54) **BODY COMPOSITION CHANGE PREDICTION DEVICE, METHOD, AND COMPUTER-READABLE STORAGE MEDIUM**

(57)     A body composition change prediction device acquires a measured acetone concentration measuring acetone excreted from a user, acquires current body composition information and past body composition information of the user, computes a prediction value predicting the body composition information at a given future point in time based on information relating to the body composition of the user, and determines a fat burning style of the user based on the acetone concentration and the current body composition information. The body composition change prediction device weights the prediction value based on a weight, the weight being set according to the burning style and being a greater weight the higher the measured ketone body concentration, and outputs information according to the weighted prediction value.

FIG.1

**Description**

BACKGROUND

Technical Field

The present invention relates to a body composition change prediction device, method, and a computer-readable storage medium.

Related Art

Japanese Patent Application Laid-Open (JP-A) No. 2016-75533 describes technology in which a fat burn rate per unit time is derived based on results of measuring a concentration of a substance contained in a biological excretion. The computed fat burn rate is then converted into an equivalent volume of food, and information is presented that expresses the equivalent volume of food.

Traditional body composition monitors are able to measure present body composition information, which reflects the past accumulation of fat mass, muscle mass, bone mass, and the like accumulated due to habitual eating patterns and/or living patterns from the past to the present. Moreover, traditional ketone body concentration measurement devices that measure the concentration of ketone bodies (acetone on the breath, acetone on the skin, ketones in the blood, ketones in urine) are also able to measure the activity level of fat metabolism resulting from increases or decreases in an energy inflow/outflow arising from a balance between a food intake amount (increasing an energy amount) and activity level (decreasing an energy amount). This enables a prediction to be made as to whether or not the body fat will decrease from now on.

A prediction of body fat change that can be determined from a ketone body concentration evaluation device as described in JP-A No. 2016-75533 is a prediction based on measurement results of the measurement subject at the current point in time, and covers a prediction for shortly thereafter, or for after passage of a comparatively short period of time (for example, for after the passage of several days). Moreover, the present inventors have found that it is difficult to use the prediction equation described in JP-A No. 2016-75533 to make a prediction for after the passage of a comparatively long period of time (for example, for after the passage of a week or more) with good precision.

SUMMARY

An object of the present invention is to provide a body composition change prediction device, method, and a computer-readable storage medium capable of predicting a change in body composition with good precision.

A body composition change prediction device of an aspect according to the present invention includes a processing circuitry configured to execute a process, the process including: acquiring, from a sensor device, a measured ketone body concentration measuring ketone bodies excreted from a user; acquiring current body composition information and past body composition information of the user; computing a prediction value predicting the body composition information at a given future point in time based on the current and past body composition information; determining a fat burning style of the user based on the measured ketone body concentration and the current body composition information; weighting the prediction value based on a weight, the weight being set according to the fat burning style and increasing as the measured ketone body concentration increases; and outputting information according to the weighted prediction value.

A second aspect according to the present invention is the first aspect, wherein the weighting calculation section computes the fat burn rate of the user based on the measured ketone body concentration, and corrects the weighted prediction value based on the computed fat burn rate.

A third aspect according to the present invention is the first or second aspect, wherein the given future point in time is after passage of a number of days in advance for prediction, and, in cases in which the number of days in advance for prediction is a predetermined threshold value or longer, the weighting calculation section corrects the weighted prediction value using a long term correction formula based on user information related to the user.

A fourth aspect according to the present invention is the third aspect, wherein the weighting calculation section corrects the weighted prediction value using a long term correction formula based on energy consumption of the user acquired from an activity level monitor.

A fifth aspect according to the present invention is any one of the first to the fourth aspects, wherein the given future point in time is after passage of a number of days in advance for prediction, and, in cases in which a period over which the past body composition information was acquired is a predetermined period or longer, the prediction value computation section computes the prediction value using a method chosen to approximate a change trend based on the past body composition information and the number of days in advance for prediction.

A sixth aspect according to the present invention is any one of the first to the fifth aspects, wherein the given future point in time is after passage of a number of days in advance for prediction, and, in cases in which a period over which the past body composition information was acquired is less than a predetermined period, the prediction value computation section computes an average value of the daily change amount of the past body composition information, and computes the prediction value based on the computed average value of the daily change amount and the number of days in advance for prediction.

A seventh aspect according to the present invention is any one of the first to the sixth aspects, wherein the given future point in time is after passage of a number of days in advance for prediction, and, in cases in which the past body composition information is not acquirable, the prediction value computation section acquires eating habit information and activity level information of the user and computes the prediction value based on the acquired eating habit information and activity level information of the user and the number of days in advance for prediction.

An eighth aspect of the present invention is a body composition change prediction method including: acquiring a measured ketone body concentration measuring ketone bodies discharged from a user; acquiring current body composition information and past body composition information of the user; computing a prediction value predicting the body composition information at a given future point in time based on the user body compositions; determining a fat burning style of the user based on the measured ketone body concentration and the current body composition information; weighting the prediction value based on a weight, the weight being set according to the burning style and being a greater weight the higher the measured ketone body concentration; and outputting information according to the weighted prediction value.

A ninth aspect of the present invention is a body composition change prediction program that causes a computer to execute processing. The processing includes: acquiring a measured ketone body concentration measuring ketone bodies discharged from a user; acquiring current body composition information and past body composition information of the user; computing a prediction value predicting the body composition information at a given future point in time based on the user body compositions; determining a fat burning style of the user based on the measured ketone body concentration and the current body composition information; weighting the prediction value based on a weight, the weight being set according to the burning style and being a greater weight the higher the measured ketone body concentration; and outputting information according to the weighted prediction value.

The aspect exhibits the advantageous effect of enabling changes to body composition to be measured with good precision.

## BRIEF DESCRIPTION OF THE DRAWINGS

Exemplary embodiments of the present invention/disclosure will be described in detail based on the following figures, wherein:

Fig. 1 is a block diagram of a body composition change prediction unit;
Fig. 2 is an external view of a sensor device;
Fig. 3 is a functional block diagram of a body composition change prediction device;
Fig. 4 is a flowchart of body composition change prediction processing;
Fig. 5 is a graph illustrating a distribution in a muscle mass index;
Fig. 6 is a diagram to explain a matrix for determining body type;
Fig. 7 is a diagram expressing images of body type;
Fig. 8 is a diagram illustrating a determination table to determine burning style;
Fig. 9 is a flowchart of a first prediction processing;
Fig. 10 is a table to explain variation width of weighting;
Fig. 11 is a flowchart of a second prediction processing; and
Fig. 12 is a flowchart of a third prediction processing.

## DETAILED DESCRIPTION

Description follows regarding exemplary embodiments of the present invention.

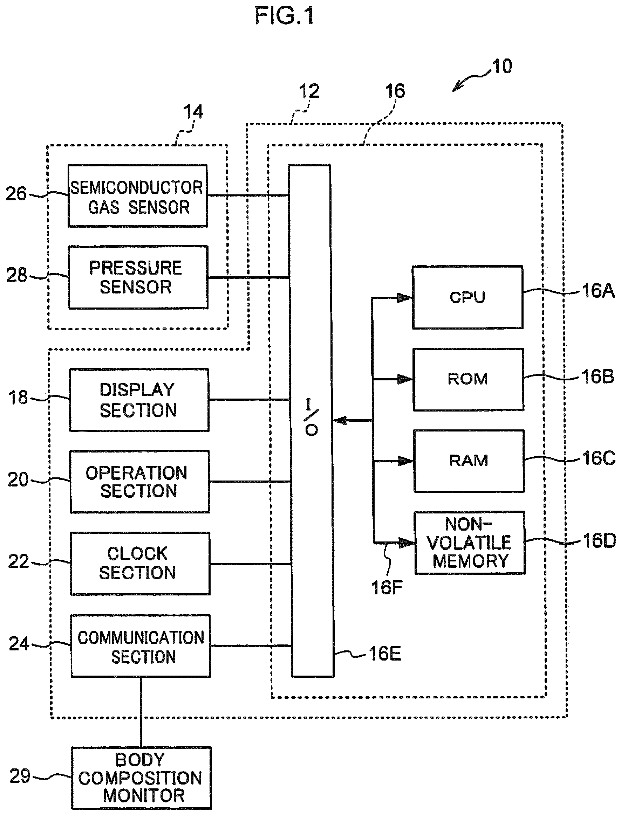

Fig. 1 is a configuration diagram of a body composition change prediction unit 10 according to an exemplary embodiment. As illustrated in Fig. 1, the body composition change prediction unit 10 includes a body composition change prediction device 12, serving as a body composition change prediction device, and a sensor device 14. The body composition change prediction device 12 includes a controller 16, a display section 18, an operation section 20, a clock section 22, and a communication section 24. The sensor device 14 includes a semiconductor gas sensor 26 and a pressure sensor 28.

The controller 16 is configured including a central processing unit (CPU) 16A, read only memory (ROM) 12B, random access memory (RAM) 16C, non-volatile memory 16D, and an input-output interface (I/O) 16E, with these being connected together through a bus 16F. In this case, a body composition change prediction program to execute body composition change prediction processing, described later, in the CPU 16A of the controller 16 is executed by, for example, writing the body composition change prediction program to the non-volatile memory 16D and reading the body composition change prediction program with the CPU 16A. Note that the body composition change prediction program may be provided on a recording medium such as a CD-ROM, a memory card, or the like, or may be downloaded from a server, not illustrated in the drawings.

The display section 18, the operation section 20, the clock section 22, the communication section 24, the semiconductor gas sensor 26, and the pressure sensor 28 are connected to the I/O 16E.

The display section 18 is, for example, configured by a liquid crystal panel or the like. Various setting screens, and various results display screens, such as of detection results are, for example, displayed on the display section 18.

The operation section 20 is an operation section for performing various operations.

Note that the display section 18 and the operation section 20 may be configured as a single unit using a touch panel, in a configuration in which operation can be performed by directly touching the touch panel.

The clock section 22 includes a function to acquire the current time, and a timing function to time durations.

The communication section 24 includes a function to exchange information with an external device such as a body composition monitor 29, either by wireless communication or wired communication.

The body composition change prediction device 12 may, for example, be a dedicated device, or may be a general purpose information processing device, such as a personal computer, a smartphone, a mobile phone, or a tablet terminal.

The semiconductor gas sensor 26 is a gas sensor having sensitivity to a biological gas such as breath blown thereon by a user. The semiconductor gas sensor 26 detects the biological gas, and outputs a voltage value according to a concentration of the detected biological gas. The biological gas in the breath contains various types of gases, such as ketone bodies, ethanol, acetaldehydes, hydrogen, water vapor, methane, and various other gases of halitosis. Ketone bodies is a general term used here to indicate at least one out of acetoacetic acid, 3-hydroxy butyric acid ($\beta$-hydroxybutyric acid), or acetone.

Specifically, the semiconductor gas sensor 26 includes a metal oxide semiconductor, such as $SnO_2$, a heater, and an electrode. The metal oxide semiconductor has a resistance value that changes when an interfering gas or an obstructing gas is adsorbed. The semiconductor gas sensor 26 lacks gas selectivity to and the ability to quantify gas, but has high sensitivity to trace quantities of acetone or the like. In the present exemplary embodiment, a case is described in which a semiconductor gas sensor is employed as a sensor to detect biological gas; however, another device, such as a gas chromatography device, may be employed to detect biological gas.

Note that in the present exemplary embodiment, a case is described in which the biological gas to be measured is acetone. Acetone is a byproduct of metabolizing fat, and the acetone concentration corresponds to the fat burn rate. Fat is not burned when there is a surplus of carbohydrate energy in the body, and so the acetone concentration is low in such cases. Fat is burned when there is insufficient carbohydrate energy in the body, and so the acetone concentration rises in such cases. This thereby enables the fat burn rate to be known from the acetone concentration.

The pressure sensor 28 detects the pressure of breath being blown thereon by a user. The pressure sensor 28 outputs the magnitude of the detected pressure as a voltage value.

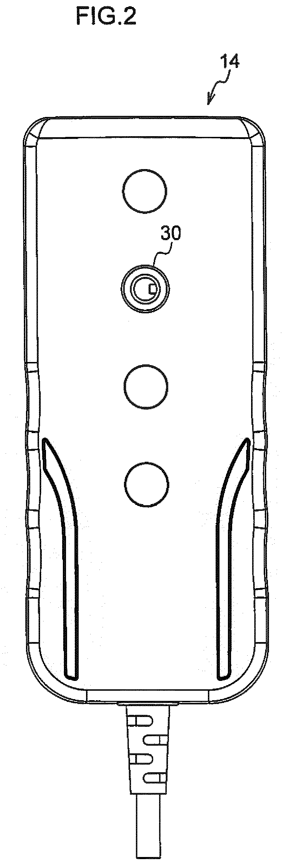

Fig. 2 is an external view of the sensor device 14. As illustrated in Fig. 2, the sensor device 14 includes a mouthpiece 30 for a user to blow breath into. When a user blows their breath into the mouthpiece 30, the biological gas is detected by the semiconductor gas sensor 26. Note that although the sensor device 14 illustrated in Fig. 2 is connected to the body composition change prediction device 12 by wire, there is no limitation thereto, and the sensor device 14 may be connected to the body composition change prediction device 12 wirelessly. Moreover, the sensor device 14 and the body composition change prediction device 12 may be formed as a single unit.

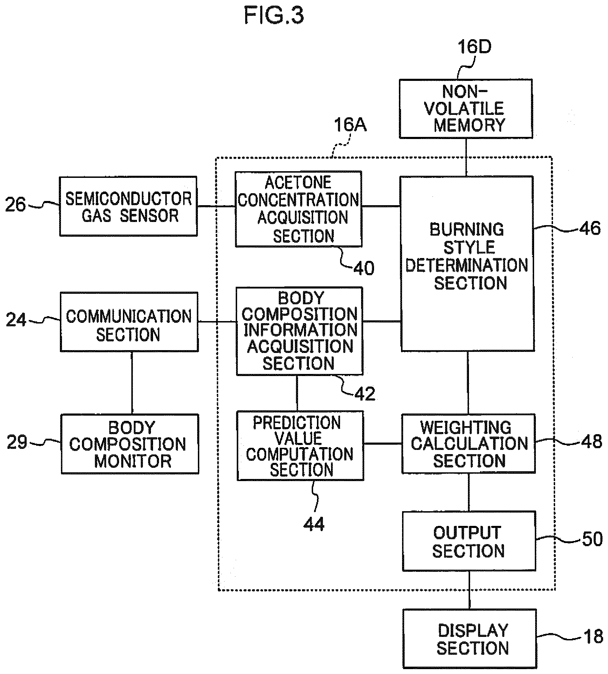

As illustrated functionally in Fig. 3, the CPU 16A of the controller 16 includes an acetone concentration acquisition section 40, a body composition information acquisition section 42, a prediction value computation section 44, a burning style determination section 46, a weighting calculation section 48, and an output section 50.

The acetone concentration acquisition section 40 serving as a ketone body concentration acquisition section acquires an acetone concentration measuring acetone excreted from a user.

The body composition information acquisition section 42 acquires current body composition information and past body composition information of a user.

Based on information related to the body composition of the user, the prediction value computation section 44 computes a prediction value predicting body composition information at a given future point in time. Note that the given future point in time is, for example, after passage of a number of days in advance for prediction, which is the number of days to pass from now until a day for which a body composition change prediction is desired (for example, 100 days), a future date (for example July 1, 2017), or the like. In the present exemplary embodiment, a case is described in which the given future point in time is after passage of a number of days in advance for prediction.

More specifically, in cases in which a period over which past body composition information was acquired is a predetermined period or longer, the prediction value computation section 44 computes a prediction value by employing a given method to approximate a change trend based on the past body composition information and the number of days in advance for prediction. Examples of the given method to approximate the change trend include a least squares method, a moving average method, or the like; however, there is no limitation thereto. In the present exemplary embodiment, a case is described in which a least squares method or the like is employed to compute the prediction value.

Based on the acetone concentration and the current body composition information, the burning style determination section 46 determines a burning style of fat of the user. Note that the burning style is an indicator that takes each body type classified by body fat percentage and muscle index, and then further classifies using fat metabolism determined from acetone concentration.

The weighting calculation section 48 weights the prediction value, based on a weight set according to the burning style such that the weight is greater the higher the acetone concentration. The weight here is an indicator of the magnitude of influence on change to body composition in the desired direction. The change to body composition in the desired direction is, for example, a reduction in body weight, and is at least one change from out of a reduction in body weight, a reduction in body fat percentage, or an increase in muscle mass. In the present exemplary embodiment, a case is described in which the greater the weight, the greater the influence on change to body composition in the desired direction.

Moreover, the weighting uses the weight to correct the prediction value (the prediction value computed using, for example, a least squares method).

The output section 50 outputs information corresponding to the prediction value arising from weighting.

Note that at least one of the acetone concentration acquisition section 40, the body composition information acquisition section 42, the prediction value computation section 44, the burning style determination section 46, the weighting calculation section 48, or the output section 50, may be configured by separate hardware, such as an application specific integrated circuit (ASIC).

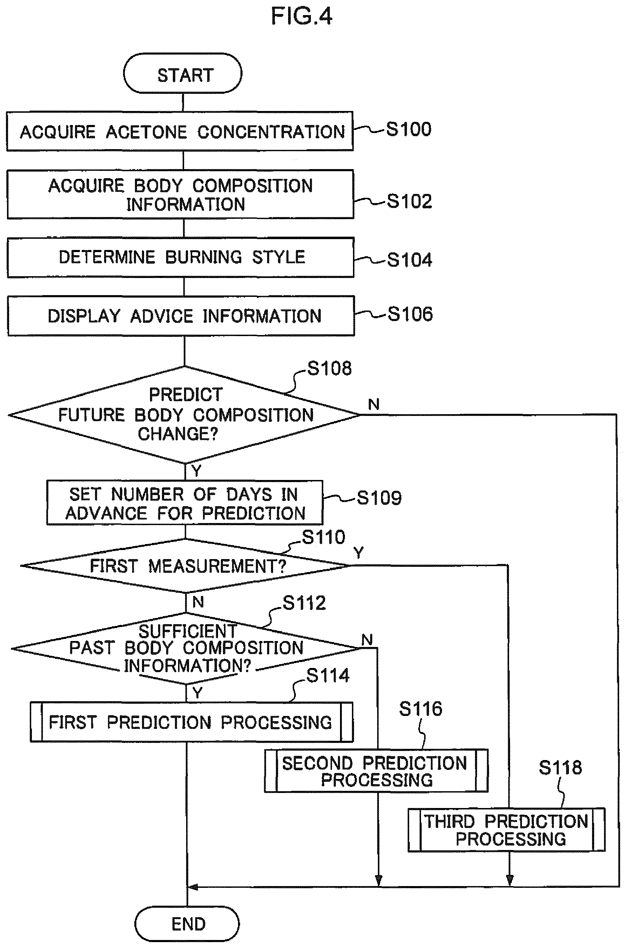

Description now follows regarding processing by a body composition change prediction program executed in the CPU 16A of the controller 16 as operation of the present exemplary embodiment, with reference to the flowchart illustrated in Fig. 4. Note that the processing illustrated in Fig. 4 is executed in cases in which execution of the body composition change prediction program has been instructed by a user operating the operation section 20 of the body composition change prediction device 12. Note that the processing illustrated in Fig. 4 may be executed on detecting blowing by the user.

At step S100, the acetone concentration acquisition section 40 displays a message on the display section 18 encouraging a user to blow a breath into the mouthpiece 30, so as to have a user blow a breath into the mouthpiece 30. The output value of the semiconductor gas sensor 26, namely, the acetone concentration $\alpha$, is then acquired. Note that many gaseous components are contained in the air of a breath immediately after starting to blow. Thus, when calculating the concentration of acetone, which is related to a fat burn rate, preferably the end of a breath, when the gaseous components in the air have been expelled, is employed. Namely, the output value of the semiconductor gas sensor 26 is preferably acquired at a timing so as to obtain the end of a breath. Thus, preferably timing by the clock section 22 is started at the point in time when blowing of a breath has been detected, and then the output value of the semiconductor gas sensor 26 is acquired at the point in time after a predetermined duration (for example, four seconds) has elapsed.

Note that determination as to whether or not a breath has been blown may, for example, be performed by acquiring an output value of the pressure sensor 28 and determining whether or not the output value acquired from the pressure sensor 28 is a predetermined threshold value or greater.

At step S102, the body composition information acquisition section 42 displays on the display section 18 a message prompting a user to measure body composition information, measures body composition information, and acquires measured body composition information from the body composition monitor 29. In the present exemplary embodiment, as an example, body weight, body fat percentage, and muscle mass are measured as body composition information. Note that body composition information input or acquired using another device, for example by using an application or the like on a smartphone, may be acquired by wireless communication or wired communication.

At step S104, the burning style determination section 46 determines the fat burning style of the user, based on an acetone concentration $\alpha$ acquired at step S100 and the body composition information acquired at step S102.

More specifically, the burning style determination section 46 computes a muscle mass index IDX according to the following equation based on a muscle mass M and a user height H from out of the body composition information acquired at step S102. The user height may be acquired from the body composition monitor 29, or may be input by the user. Note that the muscle mass index is an indicator of the amount of muscle mass based on height.

$$IDX = M/H^2 \qquad\qquad …(1)$$

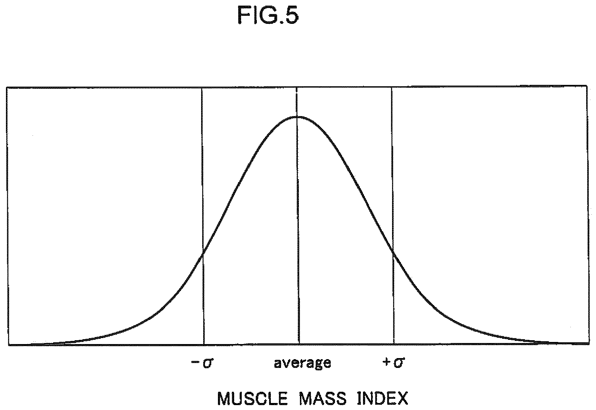

Note that although the H is employed as a square in above Equation (1), there is no limitation to H being employed as a square. Moreover, the muscle mass index IDX is, for example, a distribution as illustrated in Fig. 5.

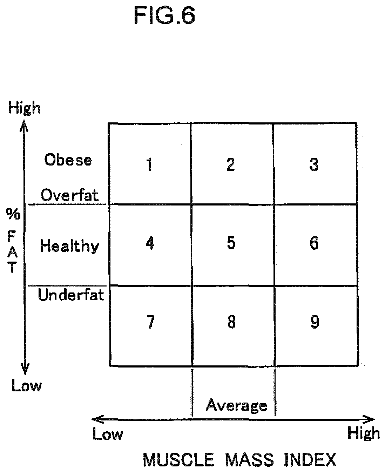

One corresponding body type is then determined from out of plural body type classifications based on the body fat percentage FAT and the muscle mass M from out of the body composition information acquired at step S102. More specifically, a matrix like that illustrated in Fig. 6 is employed, and determination is made as to which body type it is that the body type corresponding to the body fat percentage FAT and the muscle mass M of the user corresponds to from out of body types 1 to 9.

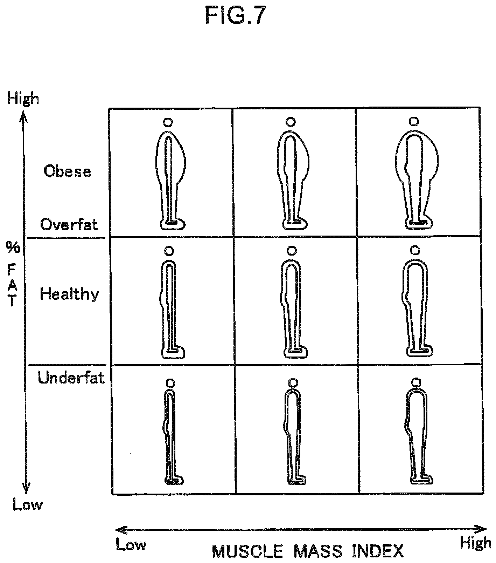

Fig. 7 is a diagram illustrating images of body types 1 to 9. As illustrated in Fig. 6 and Fig. 7, body type 1 is hidden obese type, body type 2 is obese type, body type 3 is solidly built type, body type 4 is under exercised type, body type 5 is standard type, body type 6 is muscular (1) type, body type 7 is thin type, body type 8 is thin and muscular type, and body type 9 is muscular (2) type. Note that although in the example of Fig. 6 there are nine classifications of body type, the number of body types are not limited thereto.

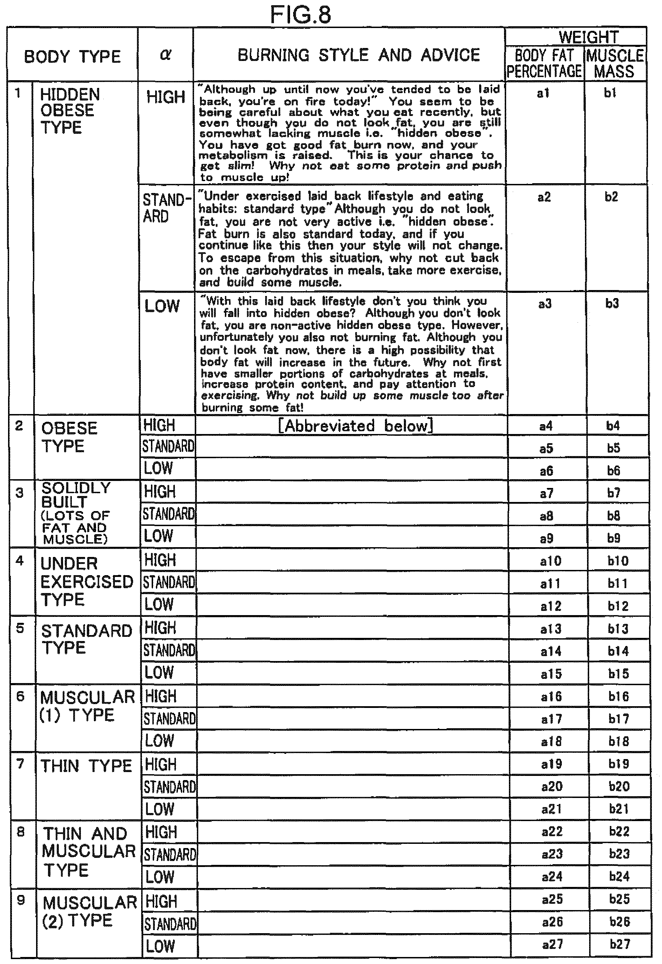

Next, the burning style is determined based on the acetone concentration $\alpha$ acquired at step S100, and the body type determined as described above. More specifically, the burning style is determined using a burning style determination table like that illustrated in Fig. 8. The burning style determination table illustrated in Fig. 8 is a table of data expressing correspondence relationships between body type, acetone concentration $\alpha$, burning style and advice, and weights (body fat percentage, muscle mass).

The acetone concentration is, as an example, divided into three levels: high, standard, and low. The burning style is determined according to the combination of body type and acetone concentration a. For example, in cases in which the acetone concentration is "high" in body type 1 (hidden obese type), the burning style is determined to be "Although up until now you've tended to be laid back, you're on fire today!".

At step S106, the output section 50 displays advice information based on the burning style determined at step S104 on the display section 18. For example, as illustrated in Fig. 8, when the burning style has been determined to be "Although up until now you've tended to be laid back, you're on fire today!", a message of "You seem to be being careful about what you eat recently, but even though you do not look fat, you are still somewhat lacking muscle i.e. "hidden obese". You have got good fat burn now, and your metabolism is raised. This is your chance to get slim! Why not eat some protein and push to muscle up!" is displayed as advice information on the display section 18.

At step S108, the prediction value computation section 44 determines whether or not to predict a future body composition change. For example, a selection screen for selecting whether or not to predict a future body composition change is displayed on the display section 18 to allow the user to select whether or not to predict a future body composition change. Then, processing transitions to step S109 when prediction of a future body composition change was selected, or the current routine is ended when prediction was not selected.

At step S109, the prediction value computation section 44 sets the number of days in advance for prediction, which is the number of days from now until a day for which a body composition change prediction is desired. Specifically, a screen to set a number of days in advance for prediction is displayed on the display section 18 to allow the user to input the number of days in advance for prediction. For example, in cases in which there is a desire to predict the body composition information in one week's time, then the number of days in advance for prediction is seven days.

At step S110, the prediction value computation section 44 determines whether or not the current time is the first measurement. For example, determination is made that the current time is the first time for measurement in cases in which there is not even a single measurement result for past body composition information (body weight, body fat percentage, and muscle mass) stored on the non-volatile memory 16D, and processing transitions to step S118. However, determination is made that the current time is not the first time for measurement in cases in which there is a measurement result for past body composition information stored on the non-volatile memory 16D, and processing transitions to step S112. Note that the current time may be determined to be the first time for measurement in cases in which, for example, it is not possible to acquire past body composition information input or acquired using another device, for example by using an application or the like on a smartphone, by wireless communication or wired communication; or determination may be made that the current time is not the first time for measurement when past body composition information can be acquired by wireless communication or wired communication.

At step S112, the prediction value computation section 44 determines whether or not measurement results for past body composition information have been being stored on the non-volatile memory 16D for a predetermined period or longer. For example, determination is made as to whether or not past body composition information for a predetermined period or longer (for example, one week's worth or longer) has been being stored on the non-volatile memory 16D. Processing transitions to step S114 in cases in which there is past body composition information stored on the non-volatile memory 16D for the predetermined period or longer, and processing transitions to step S116 in cases in which past body composition information is not stored on the non-volatile memory 16D for the predetermined period or longer, namely, when body composition information that has only been being stored for less than the predetermined period stored. Note that preferably, determination is made as to whether or not measurement results for past body composition information are stored on the non-volatile memory 16D for the predetermined period or longer and whether or not there is a predetermined number of data items stored on the non-volatile memory 16D.

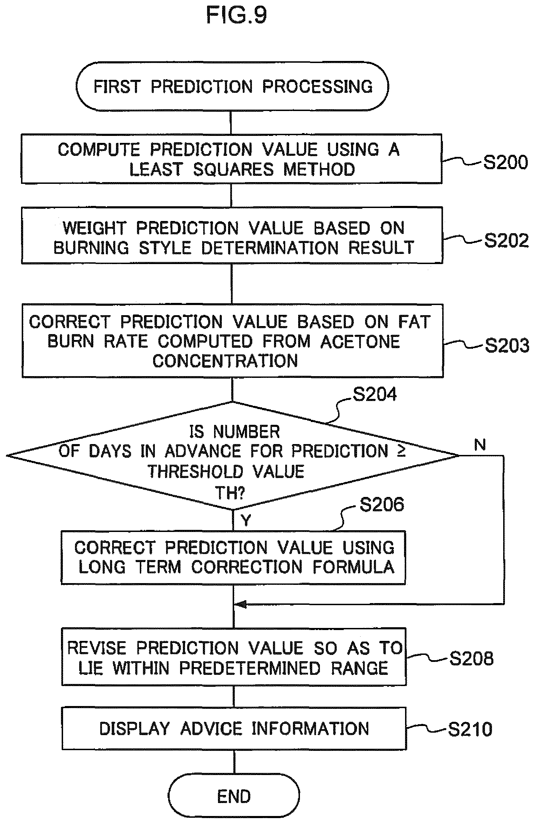

At step S114, first prediction processing illustrated in Fig. 9 is executed.

At step S200, based on the past body composition information and the number of days in advance for prediction, the prediction value computation section 44 uses a least squares method to compute a prediction value of the body composition information (body weight, body fat percentage, and muscle mass) at the number of days in advance for prediction set at step S109.

A prediction value y' is expressed by the following equation, wherein y' is a prediction value for the body composition information for a prediction after the number of days in advance for prediction, and number of days in advance for prediction is d.

$$y' = a_0 + a_1 \times d \qquad \qquad ...(2)$$

Herein, intercept $a_0$ and slope $a_1$ are obtained by solving the following simultaneous equations.

$$\sum_{i=1}^{n} y_i = na_0 + a_1 \sum_{i=1}^{n} x_i \qquad \qquad ...(3)$$

$$\sum_{i=1}^{n} x_i y_i = a_0 \sum_{i=1}^{n} x_i + a_1 \sum_{i=1}^{n} x_i^2 \qquad \qquad ...(4)$$

Wherein: $x_i$ represents the $i^{th}$ day from the start day, $y_i$ represents the body composition information on the $i^{th}$ day from the start day, and n represents the number of days the body composition information was measured in the past when the number of times the body composition information is measured is once per day. The start day is the day the oldest body composition information was measured from out of the past body composition information.

Expressing above Equations (3) and (4) in terms of the intercept $a_0$ and the slope $a_1$ gives the following equations:

$$a_0 = \frac{\sum y}{n} - a_1 \left( \frac{\sum x}{n} \right) \qquad \qquad ...(5)$$

$$a_1 = \frac{n \sum xy - \sum x \sum y}{n \sum x^2 - (\sum x)^2} \qquad \qquad ...(6)$$

Thus, in cases in which, for example, a prediction value y is desired for the body composition information at 30 days in advance using 30 days' worth of past body composition information, then days $x_1$ to $x_{30}$ from 30 days before to the current day, and the body composition information $y_1$ to $y_{30}$ from 30 days before to the current day, are substituted into above Equations (5) and (6), the intercept $a_0$ and the slope $a_1$ are computed, and the prediction value y' after d days is computed from above Equation (2).

At step S202, the weighting calculation section 48 uses a weight corresponding to the burning style determined at step S104 of Fig. 4 to weight the prediction value found at step S200. For example, for a burning style of body type "hidden obese type" and an acetone concentration of "high", as illustrated in Fig. 8, the weight for the body fat percentage is set to a1, and the weight for the muscle mass is set to b1. The prediction value for the body fat percentage is multiplied by the weight a1. Moreover, the prediction value for the muscle mass is multiplied by the weight b1.

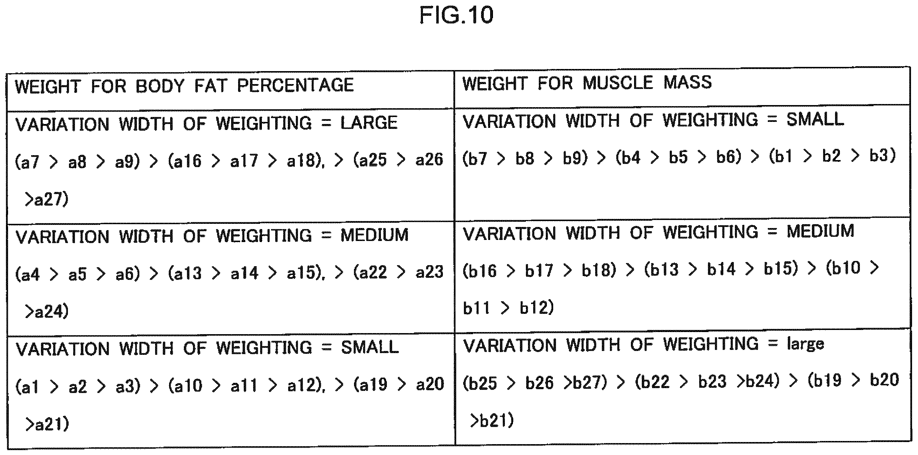

Note that, as illustrated in Fig. 10, within the same body type, the weights a1 to a27 for the body fat percentage and the weights b1 to b27 for the muscle mass increase the higher the acetone concentration of the burning style. The reason for this is to take into consideration that the rate of fat metabolism increases the higher the acetone concentration, even within the same body type. Moreover, as illustrated in Fig. 10, the weights a1 to a27 for the body fat percentage and the weights b1 to b27 for the muscle mass are classified into three levels as examples of variation width of weighting: "large", "medium", and "small".

Namely, the weight for the body fat percentage is set to a different variation width of weighting (large, medium, small) for each classification of the muscle mass index illustrated in Fig. 6 (the low side of average, average, the high side of average). Moreover, from out of the variation widths of weighting, the lowest weights (a27 for the variation width "large", a24 for the variation width "medium", and a21 for the variation width "small") are about the same magnitude ($a27 \approx a24 \approx a21$). However, from out of the variation widths of weighting, the largest weights (a7 for the variation width "large", a4 for the variation width "medium", and a1 for the variation width "small") have weights that increase as the variation width increases (a7 > a4 > a1). Note that the respective weights for the same variation width of weighting increase the larger the body fat percentage. The reason for this is to take into consideration that the greater the amount of body fat, the greater the effect of change in body composition in the desired direction due to the greater amount of body fat that can be decreased in the future.

In the example of Fig. 10, the body fat percentage is classified as "large" for weights a7 to a9, a16 to a18, and a25 to a27, "medium" for weights a4 to a6, a13 to a15, and a22 to a24, and "small" for weights a1 to a3, a10 to a12, and a19 to a21. Namely, classification is such that the higher the muscle mass index, the greater the variation width of weighting. The reason for this is to take into consideration that body fat is more easily reduced due to the basal metabolic rate increasing the greater the muscle mass.

Thus, in the weights for variation width of weighting "large", (a7 > a8 > a9) > (a16 > a17 > a18), > (a25 > a26 >a27). Moreover, in the weights for variation width of weighting "medium", (a4 > a5 > a6) > (a13 > a14 > a15), > (a22 > a23 >a24). Furthermore, in the weights for variation width of weighting "small", (a1 > a2 > a3) > (a10 > a11 > a12), > (a19 > a20 >a21). Namely, as is apparent from looking at Fig. 6, Fig. 8, and Fig. 10, the body types having the higher muscle mass index have a larger variation width of weighting, and also within the same body type, the higher the acetone concentration, the greater the weight.

Moreover, the weights for muscle mass are set with different variation widths of weighting (large, medium, small) for each of the body fat percentage classifications illustrated in Fig. 6 (the underfat side of healthy, healthy, and the overfat side of healthy). Moreover, from out of the variation widths of weighting, the lowest weights (b21 for the variation width "large", b12 for the variation width "medium", and b3 for the variation width "small") are about the same magnitude ($b21 \approx b12 \approx b3$). However, the largest weights from out of each of the variation widths of weighting (b25 for the variation width "large", b16 for the variation width "medium", and b7 for the variation width "small") increase as the attributed variation width of weighting increases (b25 < b16 < b7). Note that the respective weights increase in each of the same variation width of weighting the higher the muscle mass index. The reason for this is to take into consideration that the greater the muscle mass, the greater the effect of change in body composition in the desired direction due to the increased basal metabolism rate.

In the example illustrated in Fig. 10, muscle mass is classified as "small" for weights b7 to b9, b4 to b6, and b1 to b3, "medium" for weights b16 to b18, b13 to b15, and b10 to b12, and "large" for weights b25 to b27, b22 to b24, and b19 to b21. Namely, classification is such that the higher the body fat percentage, the smaller the variation width of weighting. The reason for this is to take into consideration that fat impedes the above effect of muscle to change the body composition in the desired direction.

In the weights for variation width of weighting "small", (b7 > b8 > b9) > (b4 > b5 > b6) > (b1 > b2 > b3). Moreover, in the weights for variation width of weighting "medium", (b16 > b17 > b18) > (b13 > b14 > b15) > (b10 > b11 > b12). Furthermore, in the weights for variation width of weighting "large", (b25 > b26 >b27) > (b22 > b23 >b24) > (b19 > b20 >b21). Namely, as is apparent from looking at Fig. 6, Fig. 8, and Fig. 10, the body types having high body fat percentage have a smaller variation width of weighting, and also within the same body type, the higher the acetone concentration, the greater the weight.

At step S203, the weighting calculation section 48 computes the daily fat burn rate based on the acetone concentration. Note that, for example, the method described in JP-A No. 2016-75533 may be employed for this computation method; however, there is no limitation thereto. Then, based on the computed fat burn rate, the prediction value that was weighted at step S202, is corrected using, for example, a predetermined correction formula or a data table.

At step S204, the prediction value computation section 44 determines whether or not the number of days in advance for prediction d is a predetermined threshold value TH (for example, several tens of days) or more. Processing transitions to step S206 when the number of days in advance for prediction d is the threshold value TH or more, and processing transitions to step S208 when the number of days in advance for prediction d is less than the threshold value TH.

At step S206, the prediction value computation section 44 uses at least one long term correction formula from out of the following predetermined first to fifth long term correction formulae, and corrects the prediction value that has been corrected at step S203.

For example, although prediction can be performed by straight line approximation for a short term body composition information prediction such as a few days or about a week in advance, linear change does not continue for long term predictions a month or more in advance, and change follows a gentle curve. Moreover, the factors resulting in the curve are various factors having a complicated relationship, and the manner in which change proceeds differs according to conditions such as the age and gender, the original physique, and the like.

For example, as body weight changes, the load acting on the body when moving varies, and the consumed energy differs even for the same action. Thus, the prediction value is corrected using the first long term correction formula that considers a change in energy consumption due to a weight change, taking body weight, serving as user information, as a parameter.

Moreover, as body weight changes, the load on the remaining fat tissue to hold a posture supporting the body changes, and the remaining fat tissue mass itself also changes. Thus, the prediction value is corrected using the second long term correction formula that considers the basal metabolism rate due to changing remaining fat tissue mass, taking body weight, serving as user information, as a parameter.

Moreover, there are also different tendencies to become overweight due to differences in lifestyle, and the effect from differences in behavioral awareness is larger as the period becomes longer. Thus, the prediction value is corrected using the third long term correction formula that considers the tendency to become overweight due to differences in lifestyle, taking energy consumption, serving as user information, as a parameter when, for example, measurement results of energy consumption, etc., can be obtained from an activity monitor, not illustrated in the drawings. Note that an activity monitor is a device capable of acquiring a number of steps, amount of exercise, an activity level, or the like, of a user.

Moreover, the trend in the change in basal metabolism rate differs with age. For example, the change in basal metabolism is different for a young person to an old person, even over the same period of time. Thus, the prediction value is corrected using the fourth long term correction formula that considers the change in the long term basal metabolism rate with age, taking age, serving as user information, as a parameter.

Moreover, the trend in body weight change with aging should be considered. Thus, the prediction value is corrected using the fifth long term correction formula that reflects a change trend in a national nutrition survey, taking age, serving as user information, as a parameter.

Note that the first to the fifth long term correction formulae are formulae derived using statistical methods.

At step S208, the prediction value computation section 44 revises the prediction value corrected at step S206 to a prediction value that lies within a predetermined range. Namely, if the prediction value exceeds a predetermined upper limit value, the prediction value is revised to the upper limit value, and if the prediction value is less than a predetermined lower limit value, the prediction value is revised to the lower limit value. The prediction value is not revised when it lies within the predetermined range. The prediction value after long term correction can thereby be prevented from being a value that is not realistic.

At step S210, the output section 50 displays advice information corresponding to the prediction value on the display section 18. For example, advice information corresponding to the prediction value is acquired from a data table expressing correspondence relationships between prediction values and advice information, and the advice information is displayed on the display section 18.

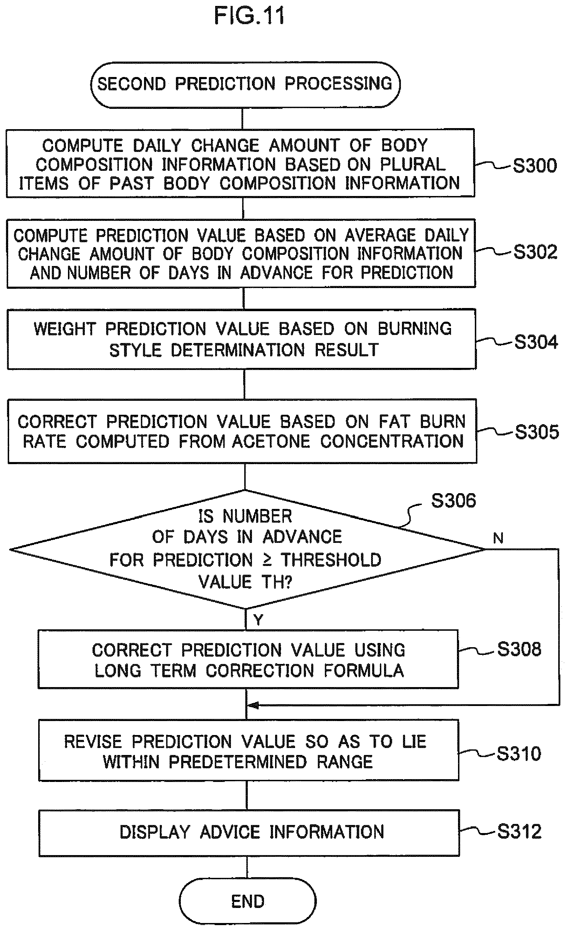

At step S116 of Fig. 4, the second prediction processing illustrated in Fig. 11 is executed.

At step S300, the prediction value computation section 44 computes a daily change amount of the body composition information based on the past body composition information. For example, when the body composition information is seven days' worth of body composition information, the daily body composition information change amount $\Delta B$ is computed according to the following equation.

$$\Delta B = \Delta W/n \qquad \qquad \ldots(7)$$

Wherein: $\Delta W$ is a change amount in the past body composition information from the oldest body composition information to the current body composition information, and n is the number of days measured for past body composition information when, as described above, the number of times the body composition information is measured is once per day.

At step S302, the prediction value computation section 44 computes a prediction value by multiplying the daily body composition information change amount $\Delta B$ computed at step S300 by the number of days in advance for prediction d.

The processing of step S304 to step S312 is similar to that of step S202 to step S210 of Fig. 9, and so explanation thereof is omitted. In this manner, when the determination result at step S112 is NO, the second prediction processing (step S116) is executed. As a result, the prediction value computation section 44 is able to compute the prediction value even in cases in which the past body composition information measurement results stored in the non-volatile memory 16D are for less than the predetermined period.

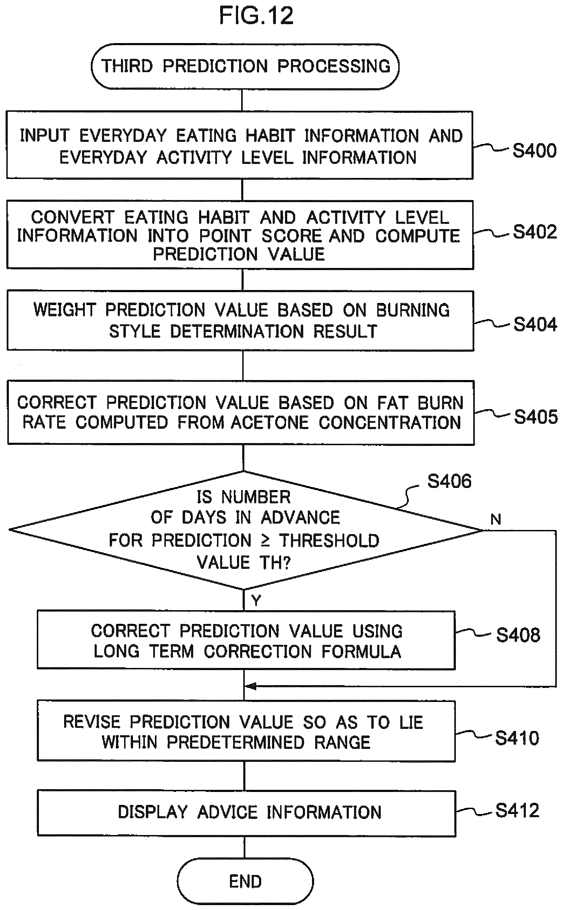

At step S118 of Fig. 4, the third prediction processing illustrated in Fig. 12 is executed.

At step S400, the prediction value computation section 44 inputs everyday eating habit information and everyday activity level information. For example, an input screen for inputting the everyday eating habit information and everyday activity level information is displayed on the display section 18, allowing the user to input the everyday eating habit information and everyday activity level information. The input screen, for example, allows a user to respond by selection in a question format. The everyday eating habit information is, for example, dietary restrictions, number of meals, meal content, food preferences, and the like. Moreover, the activity level information is, for example, an activity level itself, information that enables the activity level to be estimated, or the like.

As the eating habit information, for example, one day's worth of meal content and meal volume, and food preference information such as whether or not sweet things are eaten, are input. Moreover, as the activity level information, for example, the content and amount of exercise for one day may be input.

At step S402, the prediction value computation section 44 converts the answers that were input at step S400 to questions related to eating habit information and activity level information into a point score, and computes the prediction value. For example, a data table expressing correspondence relationships between answers to questions and points is pre-stored in the non-volatile memory 16D, and the data table is used to convert the answers to questions related to eating habit information and activity level information into a point score.

Then, the prediction value for the body composition information is computed based on the point score for the eating habit information and the point score for the activity level information. For example, either a data table or a computation formula representing correspondence relationships of eating habit information point scores and activity level point scores to body composition information is stored in the non-volatile memory 16D, and the body composition information prediction value is found using the data table or the computation formula.

The processing of step S404 to step S412 is similar to that of step S202 to step S210 of Fig. 9, and explanation thereof is omitted. In this manner, the third prediction processing (step S118) is executed when the determination result of step S110 is YES. As a result, the prediction value computation section 44 is able to compute the prediction value even in cases in which past body composition information measurement results had not been stored in the non-volatile memory 16D, namely, when the body composition information is being measured for the first time.

Note that in the first prediction processing of Fig. 9, for example, the processing of step S300 and step S302 of Fig. 11 may be executed after step S200, and a final prediction value then computed based on the prediction value computed at step S200 and the prediction value computed by executing the processing of the processing of step S300 and step S302 of Fig. 11. This enables the prediction value to be computed with good precision.

Moreover, in the first prediction processing of Fig. 9, for example, the processing of step S400 and step S402 of Fig. 12 may be executed after step S200, and a final prediction value then computed based on the prediction value computed at step S200 and the prediction value computed by executing the processing of the processing of step S400 and step S402 of Fig. 12. This enables the prediction value to be computed with good precision.

Moreover, in the second prediction processing of Fig. 11, for example, the processing of step S400 and step S402 of Fig. 12 may be executed after step S302, and a final prediction value then computed based on the prediction value computed at step S302 and the prediction value computed by executing the processing of step S400 and step S402 of Fig. 12. This enables the prediction value to be computed with good precision.

Description follows regarding specific calculation examples.

As an example, a case will be described in which body weight, body fat percentage, and muscle mass is predicted for half year in advance (180 days in advance) for a female user with a height of 160 cm, a body weight of 54 kg, and an age of 24 years.

Assume that the above female user has a current acetone concentration of 1000 ppb (somewhat high), a body fat percentage of 28% (standard), and a muscle mass of 30 kg (somewhat low), and that the past body composition information is past body composition information measured six times in the last three weeks.

In such a case, the burning style determination at step S104 of Fig. 4 is determination that the body type is "hidden obese type", and determination that the burning style is "Although up until now you've tended to be laid back, you're on fire today!" of Fig. 8.

Then, at step S106, the message "You seem to be being careful about what you eat recently, but even though you do not look fat, you are still somewhat lacking muscle i.e. "hidden obese". You have got good fat burn now, and your metabolism is raised. This is your chance to get slim! Why not eat some protein and push to muscle up!" is displayed as advice information on the display section 18.

Then, determination is made at step S108 to predict future body composition change, determination is made at step S110 that this is not measurement for the first time, the first prediction processing of step S114 is then executed when determination is made at step S112 that there is sufficient past body composition information.

In the first prediction processing, at step S200 of Fig. 9, prediction values are calculated using a least squares method according to Equation (2) for body weight, body fat percentage, and muscle mass at half a year in the future.

Then, at step S202, the weighting calculation section 48 weights by respectively multiplying the prediction value by the body fat percentage weight a1 and the muscle mass weight b1 set according to the burning style determined at step S104.

Then, at step S203 the weighting calculation section 48 computes the daily fat burn rate based on the acetone concentration. In this case the daily fat burn rate corresponding to an acetone concentration of 1000 ppb is, for example, 300 g/day. Then, the prediction value weighted at step S202 is corrected based on the computed fat burn rate.

Then, at step S206, the weighting calculation section 48 corrects the body weight, body fat percentage, and muscle mass for half a year in the future using the first to fifth long term correction formulae.

As a result, the female user is computed to have a body weight of 50 kg, a body fat percentage of 20%, and a muscle mass of 33 kg half a year in the future, for body type is determined to have a standard body type with somewhat firm muscle, and this information is displayed on the display section 18.

Moreover, a message such as, for example, "Note that this prediction result is a prediction calculated based on your body composition and fat metabolism measurement results, in consideration of statistically identified general long term change trends" may also be displayed on the display section 18.

In this manner, in the present exemplary embodiment, the fat burning style of the user is determined based on the acetone concentration and the current body composition information, and the prediction value for the future body composition information is weighted based on weights set according to the burning style. This enables the change in body composition to be predicted with good precision.

Moreover, in cases in which prediction is for a point in time beyond a given future point in time, by correcting the prediction value using the long term correction formulae, the long term body composition change can be predicted with good precision.

This enables evaluation of a "future body composition change trend" with high precision matched to individual lifestyle circumstances, and facilitates adjustment of a weight reduction target. Furthermore, being able to predict body composition change further in the future than predictions hitherto from the results of evaluating ketone body concentration alone enables lifestyle improvement advice to be presented that incorporates not only an immediate weight reduction target, but can also be easily incorporated over the longer term, and enables the utilization applications that can be enjoyed like games.

Moreover, due to the weighted prediction value being corrected based on the fat burn rate computed based on acetone concentration, body composition information reflecting the fat burn rate can be predicted with good precision.

Moreover, the weighted prediction value is corrected using the long term correction formulae based on the energy consumption of the user acquired from an activity monitor. This enables the body composition information after passage of a long period of time to be predicted with good precision.

Moreover, in cases in which the period over which the past body composition information was acquired is a predetermined period or longer, the prediction value is computed using a method chosen to approximate the change trend based on the past body composition information and the number of days in advance for prediction. This enables the body composition information to be predicted with good precision even in cases in which the body composition information changes in a non-linear manner.

Moreover, the average value of the daily change amount is computed for the past body composition information, and a prediction value is computed based on the computed average value of the daily change amount and on the number of days in advance for prediction. This enables the body composition information to be predicted even in cases in which there is not sufficient past body composition information.

Moreover, the prediction value is computed based on the eating habit information and the activity level information of the user, and on the number of days in advance for prediction. This enables the body composition information to be predicted even in cases in which there is no past body composition information.

**Claims**

1. A body composition change prediction device (12) comprising:

   a ketone body concentration acquisition section (40) that acquires, from a sensor device, a measured ketone body concentration measuring ketone bodies excreted from a user;
   a body composition information acquisition section (42) that acquires current body composition information and past body composition information of the user;
   a prediction value computation section (44) that computes a prediction value predicting the body composition information at a given future point in time based on the current and past body composition information;
   a burning style determination section (46) that determines a fat burning style of the user based on the measured ketone body concentration and the current body composition information;
a weighting calculation section (48) that weights the prediction value based on a weight, the weight being set according to the burning style and increasing as the measured ketone body concentration increases; and an output section (50) that outputs information according to the weighted prediction value.

2. The body composition change prediction device (12) of claim 1, wherein the weighting calculation section (48) computes a fat burn rate of the user based on the measured ketone body concentration, and corrects the weighted prediction value based on the computed fat burn rate.

3. The body composition change prediction device (12) of claim 1 or claim 2, wherein:

the given future point in time is after passage of a number of days in advance for prediction; and, in cases in which the number of days in advance for prediction is a predetermined threshold value or more, the weighting calculation section (48) corrects the weighted prediction value using a long term correction formula based on user information related to the user.

4. The body composition change prediction device (12) of claim 3, wherein the weighting calculation section (48) corrects the weighted prediction value using a long term correction formula based on energy consumption of the user acquired from an activity monitor.

5. The body composition change prediction device (12) of any one of claim 1 to claim 4, wherein:

the given future point in time is after passage of a number of days in advance for prediction; and, in cases in which a period over which the past body composition information was acquired is a predetermined period or longer, the prediction value computation section (44) computes the prediction value using a method chosen to approximate a change trend based on the past body composition information and the number of days in advance for prediction.

6. The body composition change prediction device (12) of any one of claim 1 to claim 5, wherein:

the given future point in time is after passage of a number of days in advance for prediction; and, the prediction value computation section (44) computes an average value of the daily change amount of the past body composition information, and computes the prediction value based on the computed average value of the daily change amount and the number of days in advance for prediction.

7. The body composition change prediction device (12) of any one of claim 1 to claim 6, wherein:

the given future point in time is after passage of a number of days in advance for prediction; and, the prediction value computation section (44) acquires eating habit information and activity level information of the user and computes the prediction value based on the acquired eating habit information and activity level information of the user and the number of days in advance for prediction.

8. A body composition change prediction method comprising:

acquiring, from a sensor device, a measured ketone body concentration measuring ketone bodies excreted from a user;
acquiring current body composition information and past body composition information of the user;
computing a prediction value predicting the body composition information at a given future point in time based on the current and past body composition information;
determining a fat burning style of the user based on the measured ketone body concentration and the current body composition information;
weighting the prediction value based on a weight, the weight being set according to the fat burning style and increasing as the measured ketone body concentration increases; and
outputting information according to the weighted prediction value.

9. A computer-readable storage medium storing a body composition change prediction program executable to cause processing circuitry to perform processing, the processing comprising:

acquiring, from a sensor device, a measured ketone body concentration measuring ketone bodies excreted from a user;

acquiring current body composition information and past body composition information of the user;

computing a prediction value predicting the body composition information at a given future point in time based on the current and past body composition information;

determining a fat burning style of the user based on the measured ketone body concentration and the current body composition information;

weighting the prediction value based on a weight, the weight being set according to the burning style and increasing as the measured ketone body concentration increases; and

outputting information according to the weighted prediction value.

# FIG.1

FIG.2

EP 3 376 505 A1

FIG.3

16

# FIG.4

```
              ( START )
                  │
    ┌─────────────────────────────┐
    │  ACQUIRE ACETONE CONCENTRATION │──── S100
    └─────────────────────────────┘
                  │
    ┌─────────────────────────────┐
    │   ACQUIRE BODY COMPOSITION    │──── S102
    │         INFORMATION           │
    └─────────────────────────────┘
                  │
    ┌─────────────────────────────┐
    │    DETERMINE BURNING STYLE    │──── S104
    └─────────────────────────────┘
                  │
    ┌─────────────────────────────┐
    │   DISPLAY ADVICE INFORMATION  │──── S106
    └─────────────────────────────┘
                  │
            S108
            ◇ PREDICT
         FUTURE BODY COMPOSITION ──── N
              CHANGE?
                  │ Y
    ┌─────────────────────────────┐
    │    SET NUMBER OF DAYS IN      │──── S109
    │   ADVANCE FOR PREDICTION      │
    └─────────────────────────────┘
                  │     S110
            ◇ FIRST MEASUREMENT? ──── Y
                  │ N      S112
            ◇ SUFFICIENT
         PAST BODY COMPOSITION ──── N
            INFORMATION?
                  │ Y      S114
    ┌─────────────────────────────┐
    │   FIRST PREDICTION PROCESSING │
    └─────────────────────────────┘

                         S116
              ┌──────────────────────┐
              │  SECOND PREDICTION    │
              │      PROCESSING       │
              └──────────────────────┘

                              S118
                  ┌──────────────────────┐
                  │   THIRD PREDICTION    │
                  │      PROCESSING       │
                  └──────────────────────┘

              ( END )
```

# FIG.5

−σ    average    +σ

MUSCLE MASS INDEX

## FIG.6

# FIG.7

## FIG.8

| BODY TYPE | | α | BURNING STYLE AND ADVICE | WEIGHT | |
|---|---|---|---|---|---|
| | | | | BODY FAT PERCENTAGE | MUSCLE MASS |
| 1 | HIDDEN OBESE TYPE | HIGH | "Although up until now you've tended to be laid back, you're on fire today!" You seem to be being careful about what you eat recently, but even though you do not look fat, you are still somewhat lacking muscle i.e. "hidden obese". You have got good fat burn now, and your metabolism is raised. This is your chance to get slim! Why not eat some protein and push to muscle up! | a1 | b1 |
| | | STAND-ARD | "Under exercised laid back lifestyle and eating habits: standard type" Although you do not look fat, you are not very active i.e. "hidden obese". Fat burn is also standard today, and if you continue like this then your style will not change. To escape from this situation, why not cut back on the carbohydrates in meals, take more exercise, and build some muscle. | a2 | b2 |
| | | LOW | "With this laid back lifestyle don't you think you will fall into hidden obese? Although you don't look fat, you are non-active hidden obese type. However, unfortunately you also not burning fat. Although you don't look fat now, there is a high possibility that body fat will increase in the future. Why not first have smaller portions of carbohydrates at meals, increase protein content, and pay attention to exercising. Why not build up some muscle too after burning some fat! | a3 | b3 |
| 2 | OBESE TYPE | HIGH | [Abbreviated below] | a4 | b4 |
| | | STANDARD | | a5 | b5 |
| | | LOW | | a6 | b6 |
| 3 | SOLIDLY BUILT (LOTS OF FAT AND MUSCLE) | HIGH | | a7 | b7 |
| | | STANDARD | | a8 | b8 |
| | | LOW | | a9 | b9 |
| 4 | UNDER EXERCISED TYPE | HIGH | | a10 | b10 |
| | | STANDARD | | a11 | b11 |
| | | LOW | | a12 | b12 |
| 5 | STANDARD TYPE | HIGH | | a13 | b13 |
| | | STANDARD | | a14 | b14 |
| | | LOW | | a15 | b15 |
| 6 | MUSCULAR (1) TYPE | HIGH | | a16 | b16 |
| | | STANDARD | | a17 | b17 |
| | | LOW | | a18 | b18 |
| 7 | THIN TYPE | HIGH | | a19 | b19 |
| | | STANDARD | | a20 | b20 |
| | | LOW | | a21 | b21 |
| 8 | THIN AND MUSCULAR TYPE | HIGH | | a22 | b22 |
| | | STANDARD | | a23 | b23 |
| | | LOW | | a24 | b24 |
| 9 | MUSCULAR (2) TYPE | HIGH | | a25 | b25 |
| | | STANDARD | | a26 | b26 |
| | | LOW | | a27 | b27 |

# FIG.9

FIRST PREDICTION PROCESSING

COMPUTE PREDICTION VALUE USING A LEAST SQUARES METHOD ⟶ S200

WEIGHT PREDICTION VALUE BASED ON BURNING STYLE DETERMINATION RESULT ⟶ S202

CORRECT PREDICTION VALUE BASED ON FAT BURN RATE COMPUTED FROM ACETONE CONCENTRATION ⟶ S203

S204
IS NUMBER OF DAYS IN ADVANCE FOR PREDICTION ≥ THRESHOLD VALUE TH?  N

Y
S206
CORRECT PREDICTION VALUE USING LONG TERM CORRECTION FORMULA

REVISE PREDICTION VALUE SO AS TO LIE WITHIN PREDETERMINED RANGE ⟶ S208

DISPLAY ADVICE INFORMATION ⟶ S210

END

FIG.10

| WEIGHT FOR BODY FAT PERCENTAGE | WEIGHT FOR MUSCLE MASS |
|---|---|
| VARIATION WIDTH OF WEIGHTING = LARGE<br><br>(a7 > a8 > a9) > (a16 > a17 > a18), > (a25 > a26<br><br>>a27) | VARIATION WIDTH OF WEIGHTING = SMALL<br><br>(b7 > b8 > b9) > (b4 > b5 > b6) > (b1 > b2 > b3) |
| VARIATION WIDTH OF WEIGHTING = MEDIUM<br><br>(a4 > a5 > a6) > (a13 > a14 > a15), > (a22 > a23<br><br>>a24) | VARIATION WIDTH OF WEIGHTING = MEDIUM<br><br>(b16 > b17 > b18) > (b13 > b14 > b15) > (b10 ><br><br>b11 > b12) |
| VARIATION WIDTH OF WEIGHTING = SMALL<br><br>(a1 > a2 > a3) > (a10 > a11 > a12), > (a19 > a20<br><br>>a21) | VARIATION WIDTH OF WEIGHTING = large<br><br>(b25 > b26 >b27) > (b22 > b23 >b24) > (b19 > b20<br><br>>b21) |

# FIG.11

```
( SECOND PREDICTION PROCESSING )
```

COMPUTE DAILY CHANGE AMOUNT OF BODY COMPOSITION INFORMATION BASED ON PLURAL ITEMS OF PAST BODY COMPOSITION INFORMATION —S300

COMPUTE PREDICTION VALUE BASED ON AVERAGE DAILY CHANGE AMOUNT OF BODY COMPOSITION INFORMATION AND NUMBER OF DAYS IN ADVANCE FOR PREDICTION —S302

WEIGHT PREDICTION VALUE BASED ON BURNING STYLE DETERMINATION RESULT —S304

CORRECT PREDICTION VALUE BASED ON FAT BURN RATE COMPUTED FROM ACETONE CONCENTRATION —S305

IS NUMBER OF DAYS IN ADVANCE FOR PREDICTION ≥ THRESHOLD VALUE TH? — S306
N

Y

CORRECT PREDICTION VALUE USING LONG TERM CORRECTION FORMULA —S308

REVISE PREDICTION VALUE SO AS TO LIE WITHIN PREDETERMINED RANGE —S310

DISPLAY ADVICE INFORMATION —S312

( END )

# FIG.12

THIRD PREDICTION PROCESSING

INPUT EVERYDAY EATING HABIT INFORMATION AND EVERYDAY ACTIVITY LEVEL INFORMATION — S400

CONVERT EATING HABIT AND ACTIVITY LEVEL INFORMATION INTO POINT SCORE AND COMPUTE PREDICTION VALUE — S402

WEIGHT PREDICTION VALUE BASED ON BURNING STYLE DETERMINATION RESULT — S404

CORRECT PREDICTION VALUE BASED ON FAT BURN RATE COMPUTED FROM ACETONE CONCENTRATION — S405

IS NUMBER OF DAYS IN ADVANCE FOR PREDICTION ≥ THRESHOLD VALUE TH? — S406

N

Y

CORRECT PREDICTION VALUE USING LONG TERM CORRECTION FORMULA — S408

REVISE PREDICTION VALUE SO AS TO LIE WITHIN PREDETERMINED RANGE — S410

DISPLAY ADVICE INFORMATION — S412

END

Europäisches
Patentamt

European
Patent Office

Office européen
des brevets

# EUROPEAN SEARCH REPORT

Application Number

EP 18 16 1519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/226747 A1 (KODAMA MIYUKI [JP] ET AL) 13 August 2015 (2015-08-13) * The whole document, in particular: Paragraphs [0010], [0012], [0013], [0034], [0044], [0050] - [0053], [0062] - [0069]; Figures 2, 7, and 8. * ----- | 1-9 | INV. G16H50/20 A61B5/00 ADD. G01N33/497 |
| X | EP 2 826 420 A1 (TANITA SEISAKUSHO KK [JP]) 21 January 2015 (2015-01-21) * The whole document, in particular: Paragraphs [0003] - [0014], [0023], [0026], [0030], [0033] - [0036], [0038], [0053], [0067] - [0069]; Figure 7. * ----- | 1-9 | |

**TECHNICAL FIELDS SEARCHED (IPC)**

G16H
A61B
G01N

The present search report has been drawn up for all claims

| Place of search | Date of completion of the search | Examiner |
|---|---|---|
| Munich | 18 June 2018 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..................................................................................

& : member of the same patent family, corresponding
    document

EPO FORM 1503 03.82 (P04C01)

## ANNEX TO THE EUROPEAN SEARCH REPORT
### ON EUROPEAN PATENT APPLICATION NO.

EP 18 16 1519

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2018

| Patent document cited in search report | | | Publication date | Patent family member(s) | | | Publication date |
|---|---|---|---|---|---|---|---|
| US 2015226747 | A1 | | 13-08-2015 | CN | 104840200 | A | 19-08-2015 |
| | | | | DE | 102015001395 | A1 | 13-08-2015 |
| | | | | JP | 6213963 | B2 | 18-10-2017 |
| | | | | JP | 2015150153 | A | 24-08-2015 |
| | | | | US | 2015226747 | A1 | 13-08-2015 |
| EP 2826420 | A1 | | 21-01-2015 | EP | 2826420 | A1 | 21-01-2015 |
| | | | | JP | 6240937 | B2 | 06-12-2017 |
| | | | | JP | 2015021744 | A | 02-02-2015 |
| | | | | US | 2015025811 | A1 | 22-01-2015 |

EPO FORM P0459

For more details about this annex : see Official Journal of the European Patent Office, No. 12/82

**EP 3 376 505 A1**

**Patent documents cited in the description**

- JP 2016075533 A